Europäisches Patentamt

European Patent Office

Office européen des brevets

(11) Publication number: **0 126 520**
**B1**

(12) **EUROPEAN PATENT SPECIFICATION**

(45) Date of publication of patent specification: **17.05.89**

(51) Int. Cl.⁴: **A 61 F 2/08**

(21) Application number: **84300875.6**

(22) Date of filing: **13.02.84**

(54) Prosthetic ligament and instruments for use in the surgical replacement of ligaments.

(30) Priority: **16.02.83 GB 8304264**

(43) Date of publication of application:
**28.11.84 Bulletin 84/48**

(45) Publication of the grant of the patent:
**17.05.89 Bulletin 89/20**

(84) Designated Contracting States:
**AT BE CH DE FR GB IT LI LU NL SE**

(56) References cited:
**DE-A-2 614 123**
**FR-A-2 135 825**
**FR-A-2 395 012**
**US-A-4 209 859**
**US-A-4 255 820**

(73) Proprietor: **Seedhom, Bahaa Botros**
**75 Holt Park Crescent**
**Leeds 16 West Yorkshire (GB)**
(73) Proprietor: **Ellis, Julian Garth**
**18 Tavistock Avenue**
**Mapperley Park Nottingham NG3 5BD (GB)**
(73) Proprietor: **Fujikawa, Kyosuke**
**Department of Orthopaedic Surgery Keio School**
**of Medicine**
**Tokyo (JP)**

(72) Inventor: **Seedhom, Bahaa Botros**
**75 Holt Park Crescent**
**Leeds 16 West Yorkshire (GB)**
Inventor: **Ellis, Julian Garth**
**18 Tavistock Avenue**
**Mapperley Park Nottingham NG3 5BD (GB)**
Inventor: **Fujikawa, Kyosuke**
**Department of Orthopaedic Surgery Keio School**
**of Medicine**
**Tokyo (JP)**

(74) Representative: **Orr, William McLean et al**
**URQUHART-DYKES & LORD 5th Floor, Tower**
**House Merrion Way**
**Leeds West Yorkshire, LS2 8PA (GB)**

Courier Press, Leamington Spa, England.

## Description

This invention relates to a new prosthetic ligament and a set of surgical instruments for use in the surgical replacement of a ligament and in particular for inserting a prosthetic ligament into the body.

In the past damaged ligaments have been replaced by grafts from other tissues of the body. However, this is not satisfactory since extensive surgery is involved and the fixation of the replacement ligament is not always adequate. Also, solid tubes of polyethylene have been used as ligaments but again the fixation of these ligaments was not satisfactory, and, further, the structure of the ligament was not suitable. Around three of four years ago carbon fibres were introduced as a possible ligament prosthesis. Holes are drilled into the bones to be joined by the ligament and multifilament strands of carbon fibres are thread through these holes, the carbon fibres being long enough to allow the strands to be knotted to fix the fibres into position. The carbon fibre proves stronger than previous materials and growth of tissue is encouraged along the length of the fibres. However, the holes which are required to be drilled in each bone are numerous and this involves large destruction of bone material which is not desirable. Further, control of the length of the ligament and its tension has proved very difficult, and anchoring to the bone has not been accepted to any degree of consistency.

One ligament which has been proposed in US—A—4255820, comprises a foraminous tube which has perforations of different size and density along its length. The perforations in the central portion of the ligament which extends between the two bones are very small in order to prevent any tissue ingrowth in this area. An internal central core is inserted into this area to help ensure that no tissue ingrowth occurs.

According to this invention there is provided a prosthetic ligament for implantation between two bones, the ligament being elongate and flexible and including two ends, each end for fixation to a bone, with a central portion of ligament extending between said ends, said central portion comprising a foraminous polyester strip characterised in that the central portion includes perforations having an area of at least 0.01 cm$^2$ so that tissue ingrowth into the central portion is promoted.

In order to simplify the manufacturing process of the ligament it is preferred that the size and distribution of the perforations in the ligament is substantially uniform along the entire length of the ligament.

The ligament may be a perforated strip of material but is preferably a fabric with an open weave structure. Preferably the polyester is polyethylene terephthalate or a copolymer thereof, for instance, the material which is marketed under the trade mark "Terylene" in the U.K. and under the trade mark "Dacron" in U.S.A. The weave of the fabric may be knitted open net but is preferably mock leno or leno woven, in which the weft threads are locked with respect to the warp threads.

Preferably there are 10—40 perforations per square cm and more preferably 15—25 perforations per cm$^2$.

Each perforation is preferably in the range of 0.01 to 0.05 cm$^2$ and is more preferably in the range of 0.01 to 0.02 cm$^2$.

Preferably such a ligament is fixed within the body by threading the prosthetic ligament through a bore in the two bones to be joined by the ligament. Each bore comprises a reduced diameter portion which extends from the point at which the original ligament was attached to the bone and an enlarged diameter portion formed by removing a plug of bone. The bone plugs are replaced into these enlarged diameter portions when the ligament has been inserted thus securing the ligament between the bone plug and the rest of the bone. The perforations in the ligament which extend through the length of the ligament promote growth of the tissue. The perforations in the region of the bone encourage bone growth which grows between the bone plug and the rest of the bone thus securing and giving a good fixation to the prosthetic ligament. The perforations in the region between the two bones encourages tissue growth along the length of the ligament which can strengthen the ligament considerably.

The ligament may be formed from a length cut from an elongate strip which is inserted in the body and then trimmed once the ligament is fixed in place. Clearly the perforations need only exist in the part of the ligament which remains in the body.

A particularly useful structure for the prosthetic ligament is for the ligament to be formed from a tube of open weave polyester. The tube includes a longitudinal slit at each end of the ligament. One of the slits extends to one end edge of the ligament. At the other end of the ligament, at the end of that slit which is remote from the centre of the ligament is a closure which seals off the tube.

When the ligament is inserted in the bone, a bone plug is inserted in the slit adjacent the seal and the other end of the ligament is pulled. This means that the bone plug is forced between the shoulder between the enlarged portion and the reduced diameter portion of the bore and the seal giving a good fixed location of the ligament. The other bone plug is then inserted through the slit at the other end of the ligament and forced against the shoulder in the other bone thus securing the ligament to both bones.

Preferably the ligament also includes means to pull the ligament through the two bores in the bones. Preferably this is in the form of a cord attached to one end of the ligament which may easily be threaded through the small bores.

Preferably the ligament is trimmed after the bone plugs have been inserted, the trimming removing the cord for threading the ligament through the bones and the sealed end of the slit.

Preferably this ligament is used to replace the cruciate ligaments or the knee. It can also, in another form, for instance a flat strip, be used to replace the medial ligament of the knee.

A prosthetic ligament according to the invention will now be described by way of example only, with reference to the accompanying drawings, in which:—

Figure 1 is a perspective view of the prosthetic ligament;

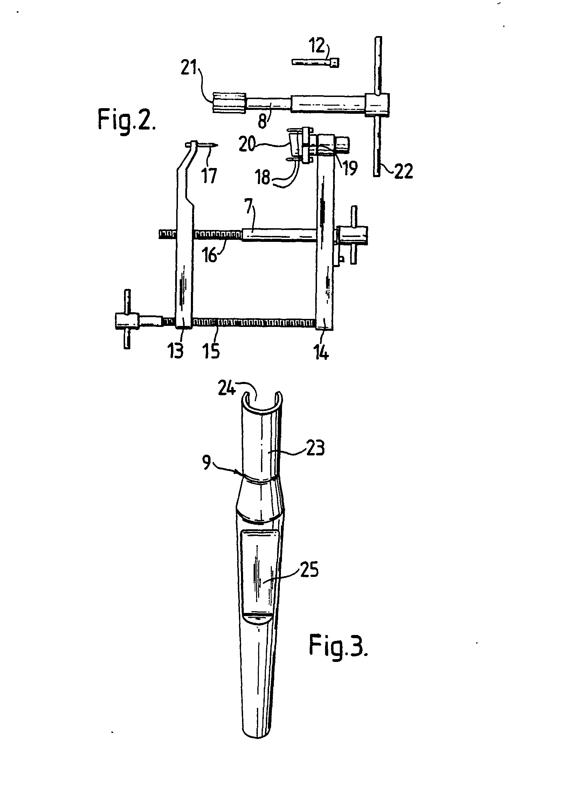

Figure 2 is a perspective view of a clamp, reamer, drill bit guide for use with the ligament of the invention;

Figure 3 is a perspective view of a bone plug extractor for use with the ligament of the invention;

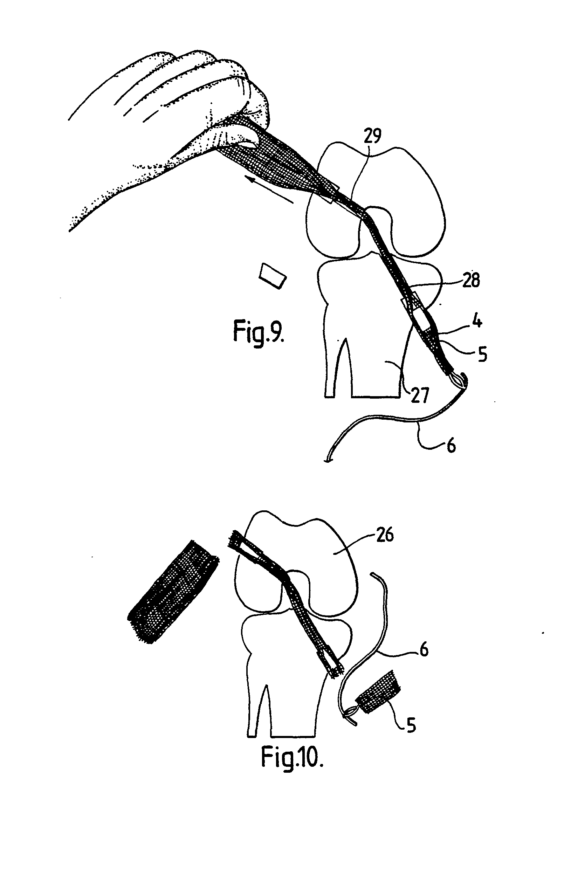

Figures 4 to 10 are schematic representations of the steps of a method of replacing an anterior cruciate ligament in the knee.

An artificial ligament 1 comprises an elongate strip of open weave polyester. This fabric is Terylene and is a mock leno weave comprising a warp of 550 Decitex polyester yarn and a weft of twisted polyester yarn. The holes are 0.1×0.5 cm. There are 14 to 15 holes across the width of the strip, and 20 holes per cm².

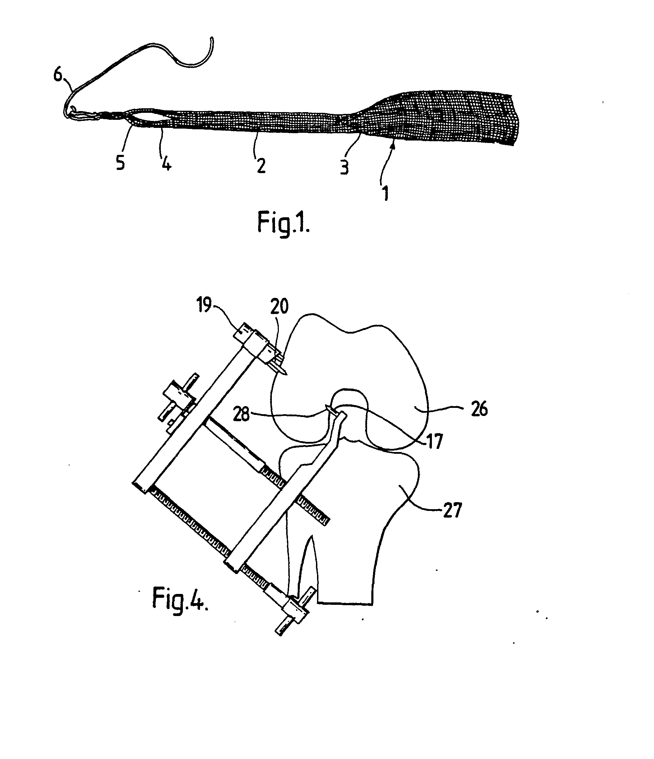

The ligament is in the form of a tube 2 which has a slit 3 at one open end of the ligament 1 which extends to the open end edge of the ligament and a slit 4 at the other closed end of the ligament. The end of the slit 4 remote from the centre of the tube 2 is sealed by a row or more of stitching 5 or a comparable method, to form a pouch. In order to illustrate the slit 4, the ligament 1 has been shown in Figure 1 with a dark coloured tubular insert. This means that the pouch-like structure is not clearly illustrated. A cord 6 is attached to the end of the ligament to pull the ligament through the bores in the bone.

A set of surgical instruments for use in the implantation of the ligament comprises a clamp 7, a reamer 8, a bone plug extractor 9, and a drill bit guide 12.

The clamp 7 comprises two parallel limbs 13, 14 connected by two perpendicular screw threaded members 15 and 16. One limb includes a first guide pin 17 standing perpendicularly of the limb. The other limb 14 includes two guide pins 18 which are parallel to the guide pin 17 and extend towards the guide pin 17. Also connected to limb 14 is a cylindrical guide 19 which has a surface 20 between the two limbs which is tilted, the two guide pins are screwed into the cylindrical guide 19.

The reamer 8 passes through the cylindrical guide 19 and has sharp cutting edges 21, which are milled along its surface and form sharp teeth at the rim of the reamer, and a handle 22 which may be used to oscillate the cuting edges 21 to remove the bone dust.

The bone plug extractor 9 comprises a cylindrical portion 23 which includes a cut-out portion 24. The bone plug extractor 9 has a flattened portion 25 on its handle. The diameter of the cylindrical portion 23 is the same as the reamer.

The drill bit guide 12 is placed in the cylindrical hole created in the bone and is used as a guide for the drill bit to drill out the reduced diameter portion of the bore.

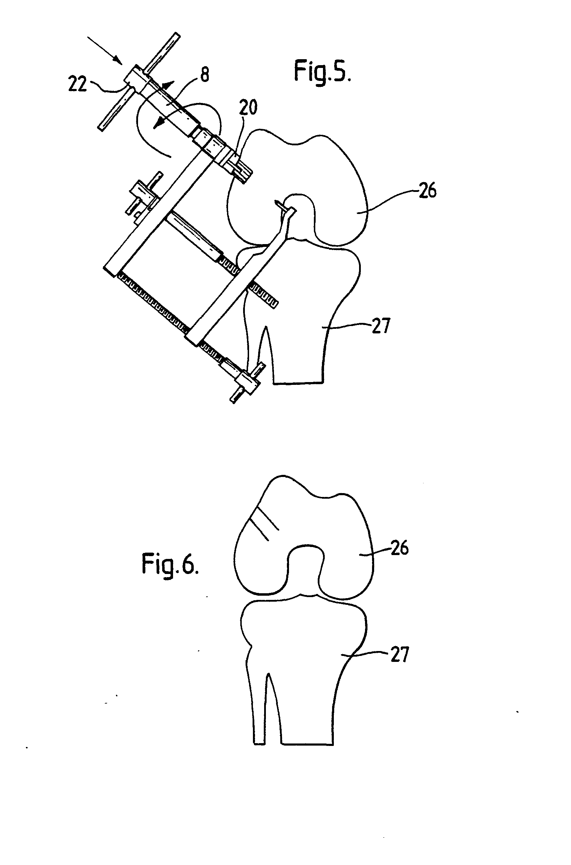

The anterior cruciate ligament in the knee is connected to the femur 26 and the tibia 27. As shown in Figure 4, the clamp 7 is placed around the femur 26 with the first guide pin 17 placed at the point of attachment 28 where the original ligament was attached to the femur 26. The clamp is then rotated until the sloping edge 20 of the cylindrical guide 19 is aligned to the slope of the bone 26. The clamp 7 is then tightened up locating the axis of the bore.

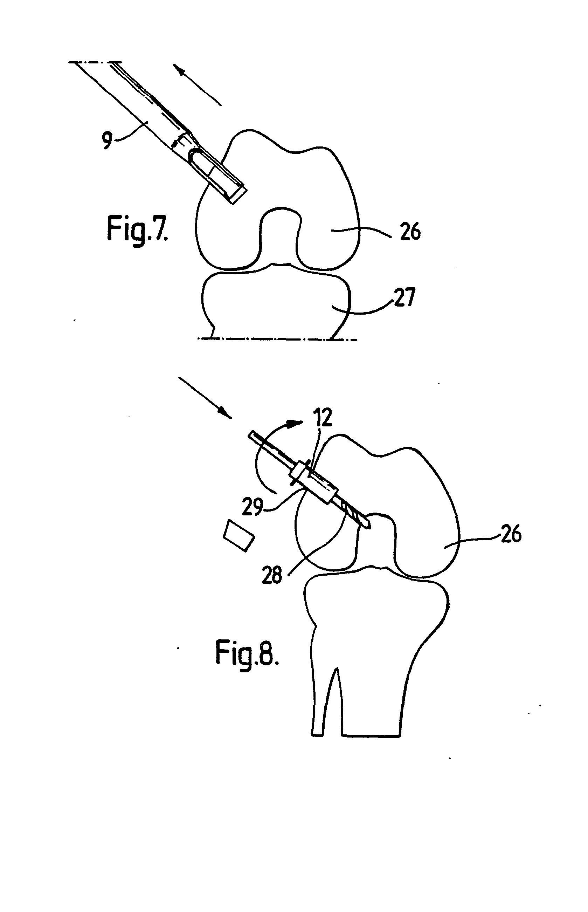

As shown in Figure 5 the reamer 8 is then placed within the cylindrical guide 20 and the handle 22 is oscillated to remove an annulus of bone dust (Figure 6). The reamer is then removed from the bone. In the infrequent possibility that the bone plug is removed with the reamer, a rod is inserted into the reamer to remove the bone plug.

As shown in Figure 7 the bone plug extractor 9 is then placed in the annulus defined in the femur 26. A swift force is applied to the flattened surface 25 to remove the bone plug from the bone, whilst the bone plug extractor 9 is gripped firmly. The bone plug may be removed from the bone plug extractor 9 by inserting a rod into opening 24.

The drill bit guide 12 is then placed in the cylindrical hole left in the femur 26 and a drill bit is then placed within the guide 12 to drill out the reduced diameter portion 28 of the bore 29 now formed through the femur 26.

This procedure is then repeated in the tibia forming a bore in the tibia. The ligament 1 is the threaded through the two bores 29 using the cord 6. The bone plug from the tibia 27 is then placed within the slit 4 in the ligament.

The other end of the ligament 1 is then pulled forcing the bone plug against the shoulder formed between the enlarged portion and the reduced diameter portion 28 by the sealed end 5.

The bone plug from the femur 26 is then forced into the opening 3, aided by a push rod. Thus the ligament 1 is secured to both the femur and tibia.

Subsequently the anchorage will be strengthened as a result of the bone growth between the bone plug and the rest of the bone. Also tissue growth is encouraged along the length of ligament between the bones thus strengthening the ligament.

**Claims**

1. A prosthetic ligament for implantation between two bones, the ligament being elongate and flexible and including two ends, each end for fixation to a bone, with a central portion of ligament extending between said ends, said central portion comprising a foraminous polyester strip characterised in that the central portion includes perforations having an area of at least 0.01 cm² so that tissue ingrowth into the central portion is promoted.

2. A prosthetic ligament according to claim 1, further characterised in that the entire ligament is foraminous and has perforations the size and distribution of which are substantially uniform along the length of the ligament.

3. A prosthetic ligament according to claim 1, further characterised in that the ligament comprises a polyester fabric with an open weave structure.

4. A prosthetic ligament according to claim 3, further characterised in that the polyester is polyethylene terephthalate or a copolymer thereof.

5. A prosthetic ligament according to any one of the claims 2 to 4, further characterised in that the weave of the fabric is mock leno or leno woven in which the weft threads are locked with respect to the warp threads.

6. A prosthetic ligament according to any one of the preceding claims, further characterised in that the distribution of the perforations is in the range 10 to 40 perforations/cm$^2$.

7. A prosthetic ligament according to claim 6, further characterised in that the distribution of the perforations is in the range 15 to 25 perforations/cm$^2$.

8. A prosthetic ligament according to any one of the preceding claims, further characterised in that the size of the perforations is in the range 0.01 to 0.05 cm$^2$.

9. A prosthetic ligament according to claim 8, further characterised in that the size of the perforations is in range of 0.01 to 0.02 cm$^2$.

10. A prosthetic ligament according to any one of the preceding claims, further characterised in that the ligament (1) is tubular, the tube including no core.

11. A prosthetic ligament according to claim 10, further characterised in that the tube (2) has a first open end and a second closed end (5) and that a first longitudinal slit (3) extends from the first end to a point close to but spaced from the first end, and a second longitudinal slit (4) is situated adjacent the second closed end.

12. A prosthetic ligament according to claim 11, further characterised in that it includes means to pull the ligament.

13. A prosthetic ligament according to claim 12, further characterised in that the means comprises a cord attached to one end of the ligament.

14. A prosthetic ligament according to Claim 11, characterised in that the second slit (4) defines the entrance of a pouch for receiving a bone plug.

## Patentansprüche

1. Prothetisches Ligament zur Implantation zwischen zwei Knochen, das langgestreckt und flexibel ist und zwei Enden aufweist, die jeweils an einem Knochen befestigbar sind, mit einem zentralen Ligamentbereich, der zwischen den genannten Enden liegt und einen gelochten Polyesterstreifen aufweist, dadurch gekennzeichnet, daß der zentrale Bereich Perforierungen mit einer Flächengröße von wenigstens 0,01 cm$^2$ besitzt, so daß das Einwachsen von Gewebe in den zentralen Bereich begünstigt wird.

2. Prothetisches Ligament nach Anspruch 1, dadurch gekennzeichnet, daß das gesamte Ligament gelocht ist und Perforationen mit über die Länge des Ligaments im wesentlichen gleichförmiger Größe und Verteilung besitzt.

3. Prothetisches Ligament nach Anspruch 1, dadurch gekennzeichnet, daß es ein Polyestergewebe mit offener Webstruktur aufweist.

4. Prothetisches Ligament nach Anspruch 3, dadurch gekennzeichnet, daß das Polyester Polyäthylenterephtalat oder ein Kopolymer hiervon ist.

5. Prothetisches Ligament nach einem der Ansprüche 2 bis 4, dadurch gekennzeichnet, daß die Bindung des Gewebes Scheindreherbindung oder Dreherbindung ist, in welchem die Schußfäden gegenüber den Kettfäden festgelegt sind.

6. Prothetisches Ligament nach einem der vorhergehenden Ansprüche, dadurch gekennzeichnet, daß die Perforierungsverteilung im Bereich von 10 bis 40 Perforationen pro cm$^2$ liegt.

7. Prothetisches Ligament nach Anspruch 6, dadurch gekennzeichnet, daß die Perforierungsverteilung im Bereich von 15 bis 25 Perforationen pro cm$^2$ liegt.

8. Prothetisches Ligament nach einem der vorhergehenden Ansprüche, dadurch gekennzeichnet, daß die Größe der Perforationen im Bereich von 0,01 bis 0,05 cm$^2$ liegt.

9. Prothetisches Ligament nach Anspruch 8, dadurch gekennzeichnet, daß daß die Größe der Perfortionen im Bereich von 0,01 bis 0,02 cm$^2$ liegt.

10. Prothetisches Ligament nach einem der vorhergehenden Ansprüche, dadurch gekennzeichnet, daß das Ligament (1) rohrförmig ist, wobei das Rohr keinen Kern besitzt.

11. Prothetisches Ligament nach Anspruch 11, dadurch gekennzeichnet, daß das Rohr (2) ein erstes offenes Ende und ein zweites geschlossenes Ende besitzt und daß ein erster Längsschlitz (3) von dem ersten Ende bis zu einem Punkt verläuft, der sich in der Nähe des ersten Endes, jedoch im Abstand von diesem befindet, und ein zweiter Längsschlitz (3) in der Nähe des geschlossenen zweiten Endes liegt.

12. Prothetisches Ligament nach Anspruch 11, dadurch gekennzeichnet, daß es Mittel zum Ziehen des Ligaments aufweist.

13. Prothetisches Ligament nach Anspruch 12, dadurch gekennzeichnet, daß diese Mittel einen an einem Ende des Ligaments befestigte Schnur umfassen.

14. Prothetisches Ligament nach Anspruch 11, dadurch gekennzeichnet, daß der zweite Schlitz (4) den Eingang für eine Tasche zur Aufnahme eines Knochen-Dübels bildet.

## Revendications

1. Ligament artificiel destiné à être implanté entre deux os, le ligament étant allongée et souple et comportant deux extrémités, chaque extrémité étant destinée à être fixée à un os, une partie centrale de ligament s'étendant entre lesdites extrémités et comprenant une bande foraminée en polyester, caractérisé en ce que la partie centrale comporte des perforations ayant une surface d'au moins 0,01 cm² de sorte que la croissance de tissu cellulaire à l'intérieur de la partie centrale soit favorisée.

2. Ligament artificiel suivant la revendication 1, caractérisé en outre en ce que le ligament entier est foraminé et comporte des perforations dont la dimension et la distribution sont sensiblement uniformes sur la longueur du ligament.

3. Ligament artificiel suivant la revendication 1, caractérisé en outre en ce qu'il comporte un tissu en polyester ayant une structure d'armure ouverte.

4. Ligament artificiel suivant la revendication 3, caractérisé en outre en ce que le polyester est un polyéthylène-téréphthalate ou un copolymère de celui-ci.

5. Ligament artificiel suivant l'une quelconque des revendications 2 à 4, caractérisé en outre en ce que l'armure de tissu est une armure de gaze ou de gaze imitée dans laquelle les fils de trame sont immobilisés par rapport au fil de chaîne.

6. Ligament artificiel suivant l'une quelconque des revendications précédentes, caractérisé en outre en ce que la distribution des perforations est dans la plage de 10 à 40 perforations/cm².

7. Ligament artificiel suivant la revendication 6, caractérisé en outre en ce que le distribution des perforations est dans la plage de 15 à 25 perforations/cm².

8. Ligament artificiel suivant l'une quelconque des revendications précédentes, caractérisé en outre en ce que la dimension des perforations est dans la plage de 0,01 à 0,05 cm².

9. Ligament artificiel suivant la revendication 8, caractérisé en outre en ce que la dimension des perforations est dans la plage de 0,01 à 0,02 cm².

10. Ligament artificiel suivant l'une quelconque des revendications précédentes, caractérisé en outre en ce que le ligament (1) est tubulaire et que le tube ne comporte pas de noyau.

11. Ligament artificiel suivant la revendication 10, caractérisé en outre en ce que le tube (2) a une première extrémité ouverte et une seconde extrémité fermée (5) et en ce qu'une première fente longitudinale (3) s'étend à partir de la première extrémité jusqu'en un point à proximité mais espacé de la première extrémité, et en ce qu'une seconde fente longitudinale (4) est disposée adjacente à la seconde extrémité fermée.

12. Ligament artificiel suivant la revendication 11, caractérisé en outre en ce qu'il comporte des moyens d'extraction du ligament.

13. Ligament artificiel suivant la revendication 12, caractérisé en outre en ce que les moyens comportent une corde fixée à l'une des extrémités du ligament.

14. Ligament artificiel suivant la revendication 11, caractérisé en outre en ce que la seconde fente (4) définit l'entrée d'un sachet destiné à recevoir une cheville en os.

Fig.1.

Fig.4.

Fig.2.

Fig.3.

22    8    20    Fig.5.

26

27

Fig.6.    26

27

Fig.7.

9

26

27

Fig.8.

12

29

28

26

Fig.9.

Fig.10.